# EUROPEAN PATENT APPLICATION

(11) **EP 3 225 278 A1**
(43) Date of publication of application: **04.10.2017**
(21) Application number: 17163293.8
(22) Date of filing: 28.03.2017
(51) Int. Cl.: A61N 1/05, A61N 1/36

(54) **DISTRIBUTED ELECTRODE LEAD CONFIGURATIONS AND ASSOCIATED SYSTEMS AND METHODS**

(30) Priority: 30.03.2016 US 201662315476 P; 15.03.2017 US 201715460064
(71) Applicant: Nevro Corp., Redwood City, CA 94065 (US)
(72) Inventor: THACKER, James R, Redwood City, CA 94065 (US)
(74) Representative: Hoarton, Lloyd Douglas Charles

(57) **Abstract**

Distributed electrode lead configurations for providing electrical therapy, and associated systems and methods. A representative patient therapy system includes at least one implantable signal delivery device having a proximal portion and a distal portion. The proximal portion is configured to be coupled with an implantable pulse generator to direct a pulsed electrical signal at a frequency from about 1.5 kHz to about 100 kHz. The distal portion includes multiple electrical contacts for delivering the pulsed electrical signal to a target neural population of a patient when implanted. At least three neighboring electrical contacts of the multiple electrical contacts are electrically connected together and have equal lengths less than 3 mm. The at least three neighboring electrical contacts are uniformly spaced apart by a distance from about 1 mm to about 8 mm.

## Description

### CROSS-REFERENCE TO RELATED APPLICATION

The present application claims priority to pending U.S. Provisional Patent Application No. 62/315,476, filed on March 30, 2016 and incorporated herein by reference.

### TECHNICAL FIELD

The present technology is directed generally to distributed electrode lead configurations for providing electrical therapy, and associated systems and methods.

### BACKGROUND

Neurological stimulators have been developed to treat pain, movement disorders, functional disorders, spasticity, cancer, cardiac disorders, and various other medical conditions. Implantable neurological stimulation systems generally have an implantable signal generator (sometimes referred to as an "implantable pulse generator" or "IPG") and one or more leads that deliver electrical pulses to neurological tissue or muscle tissue. For example, several neurological stimulation systems for spinal cord stimulation (SCS) have cylindrical leads that include a lead body with a circular cross-sectional shape and one or more conductive rings spaced apart from each other at the distal end of the lead body. The conductive rings operate as individual electrodes and, in many cases, the SCS leads are implanted percutaneously through a large needle inserted into the epidural space, with or without the assistance of a stylet.

Once implanted, the signal generator applies electrical signals to the electrodes, which in turn modify the function of the patient's nervous system, such as by altering the patient's responsiveness to sensory stimuli and/or altering the patient's motor-circuit output. In SCS therapy for the treatment of pain, the signal generator applies electrical signals to the electrodes. In conventional SCS therapy, electrical pulses are used to generate sensations (known as paresthesia) that mask or otherwise alter the patient's sensation of pain. For example, in many cases, patients report a tingling or paresthesia that is perceived as more pleasant and/or less uncomfortable than the underlying pain sensation.

In contrast to traditional or conventional (i.e., paresthesia-based) SCS, a form of paresthesia-free SCS has been developed that uses therapy signal parameters that treat the patient's sensation of pain without generating paresthesia or otherwise using paresthesia to mask the patient's sensation of pain. One of several advantages of paresthesia-free SCS therapy systems is that they eliminate the need for uncomfortable paresthesias, which many patients find objectionable. While the foregoing stimulators and techniques have proven beneficial in many instances, there remains a significant need for improved techniques and systems for addressing patient pain in a manner that is effective and comfortable over an extended period of time.

### BRIEF DESCRIPTION OF THE DRAWINGS

Figure 1A is a partially schematic illustration of an implantable spinal cord stimulation system positioned at the spine to deliver therapeutic signals in accordance with an embodiment of the present technology.
Figure 1B is a partially schematic illustration of the general layout of a lead for use with the stimulation system of Figure 1A, in accordance with an embodiment of the present technology.
Figure 2A is a top view of a lead having electrode contacts configured in accordance with an embodiment of the present technology.
Figure 2B is a top view of a lead having electrode contacts configured in accordance with another embodiment of the present technology.

### DETAILED DESCRIPTION

### 1.0 Introduction

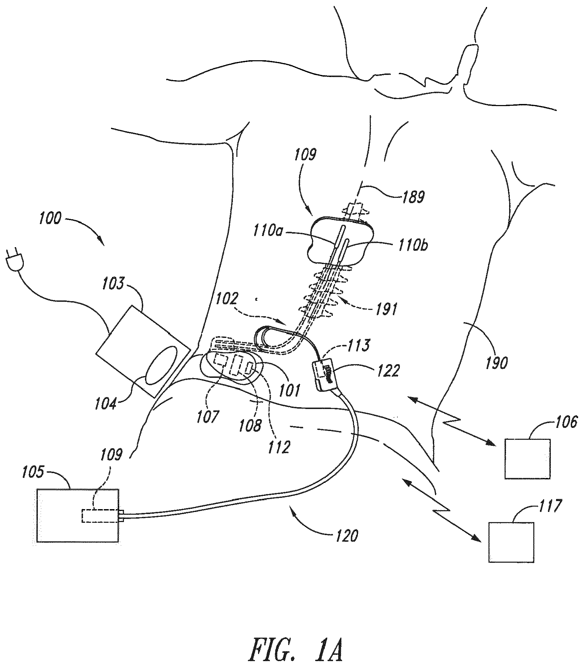

The present technology is directed generally to distributed electrode lead configurations for providing electrical therapy, and associated systems and methods. Specific details of certain embodiments of the present technology are described below with reference to a distribution of electrodes on a lead for spinal cord modulators, which can be used to treat chronic pain. For example, in a representative embodiment, a lead includes at least four neighboring electrical contacts grouped together and electrically connected using a common conductor. Each of the contacts can have a length of about 0.75 mm. The contacts of the group of neighboring electrical contacts can be uniformly spaced apart by a distance of about 1 mm. However, in other embodiments, the contacts of the group of neighboring electrical contacts can be spaced apart by different or non-uniform distances. The lead can be coupled to an implantable pulse generator programmed to direct a pulsed electrical signal to a patient at a frequency from about 1.5 kHz to about 100 kHz via the contacts. However, the disclosed systems and methods can be used in the context of other modulators and/or other patient conditions. Accordingly, some embodiments can have configurations, components, or procedures different than those described in this section, and other embodiments can eliminate particular components or procedures. For example, in some embodiments, three neighboring electrical contacts can be grouped together and electrically connected using a common conductor. The present technology can include other embodiments with additional elements, and/or can include other embodiments without several of the features shown and described below with reference to Figures 1A-2B.

As used herein, unless explicitly stated otherwise, the terms "pulses" and "signals" include any suitable waveform shape, whether continuous or discontinuous, including but not limited to sinusoidal or non-sinusoidal waves such as truncated exponential square waves, triangle waves, sawtooth waves, etc.

For parameters described herein that are modified by the term "about," the term "about" refers to a margin of plus or minus 5%. Any parameter modified by the term "about" can in other embodiments can include the exact value.

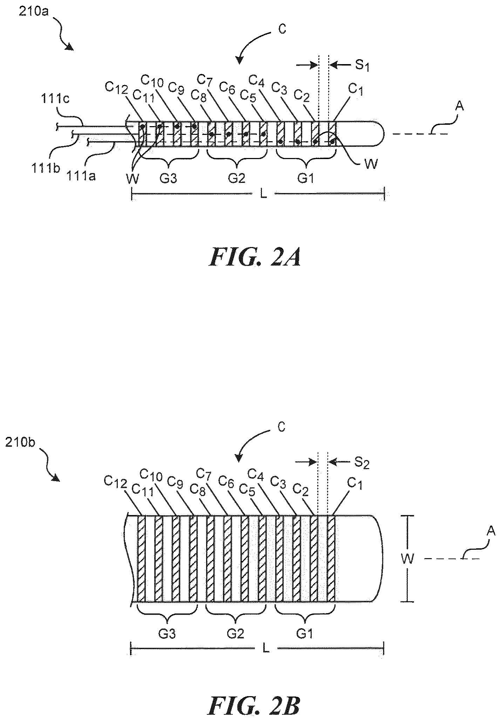

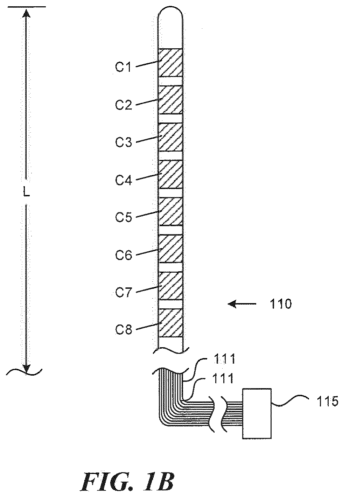

In the following discussion, Figure 1A illustrates a representative implementation of a system implanted in the patient's spinal cord region. Figure 1 B illustrates a general arrangement of a lead in accordance with the present technology, with further details described with respect to Figures 2A-2B. Figures 2A-2B illustrate representative lead configurations configured in accordance with embodiments of the present technology for use with a system such as is illustrated Figure 1A.

Figure 1A schematically illustrates a representative patient therapy system 100 for providing relief from chronic pain and/or other conditions, arranged relative to the general anatomy of a patient's spinal column 191. The system 100 can include a signal generator 101 (e.g., an implanted or implantable pulse generator or IPG), which can be implanted subcutaneously within a patient 190 and coupled to one or more signal delivery elements or devices 109. The signal delivery elements or devices 109 may be implanted within the patient 190, typically at or near the patient's spinal cord midline 189. The signal delivery devices 109 carry features for delivering therapy to the patient 190 after implantation. The signal generator 101 can be connected directly to the signal delivery devices 109, or it can be coupled to the signal delivery devices 109 via a signal link or lead extension 102. In a further representative embodiment, the signal delivery devices 109 can include one or more elongated lead(s) or lead body or bodies 110 (identified individually as a first lead 110a and a second lead 110b). As used herein, the terms signal delivery device, lead, and/or lead body include any of a number of suitable substrates and/or support members that carry devices for providing therapy signals to the patient 190. For example, the lead or leads 110 can include one or more electrodes or electrical contacts that direct electrical signals into the patient's tissue, such as to provide for patient relief. In other embodiments, the signal delivery devices 109 can include structures other than a lead body (e.g., a paddle) that also direct electrical signals and/or other types of signals to the patient 190. Cylindrical leads or paddle leads are representative examples of signal delivery devices.

In a representative embodiment, one signal delivery device may be implanted on one side of the spinal cord midline 189, and a second signal delivery device may be implanted on the other side of the spinal cord midline 189. For example, the first and second leads 110a, 110b shown in Figure 2A may be positioned just off the spinal cord midline 189 (e.g., about 1 mm offset) in opposing lateral directions so that the two leads 110a, 110b are spaced apart from each other by about 2 mm. In particular embodiments, the leads 110 may be implanted at a vertebral level ranging from, for example, about T8 to about T12. In other embodiments, one or more signal delivery devices can be implanted at other vertebral levels, e.g., as disclosed in U.S. Patent Application Publication No. 2013/0066411, which is incorporated herein by reference in its entirety.

The signal generator 101 can transmit signals (referred to as electrical signals or therapy signals) to the signal delivery devices 109 that up-regulate (e.g., stimulate or excite) and/or down-regulate (e.g., block, inhibit, or suppress) target nerves. As used herein, and unless otherwise noted, to "stimulate," "modulate," or provide "stimulation" or "modulation" to the target nerves refers generally to producing either type of the foregoing effects on the target nerves. The signal generator 101 can include a machine-readable (e.g., computer-readable) medium or controller-readable medium containing instructions for generating and transmitting suitable therapy signals. The signal generator 101 and/or other elements of the system 100 can include one or more processor(s) 107, memory unit(s) 108 and/or input/output device(s) 112. Accordingly, the process of providing modulation signals, providing guidance information for locating or positioning the signal delivery devices 109, determining impedance and taking follow-up action, and/or executing other associated functions can be performed by computer-executable instructions contained on computer-readable media located at the signal generator 101 and/or other system components, e.g., at the processor(s) 107 and/or memory unit(s) 108. The signal generator 101 can include multiple portions, elements, and/or subsystems (e.g., for directing signals in accordance with multiple signal delivery parameters), housed in a single housing, as shown in Figure 1A, or in multiple housings.

The signal generator 101 can also receive and respond to an input signal received from one or more sources. The input signals can direct or influence the manner in which the therapy, charging, and/or process instructions are selected, executed, updated, and/or otherwise performed. The input signals can be received from one or more sensors (e.g., an input device 112 shown schematically in Figure 2A for purposes of illustration) that are carried by the signal generator 101 and/or distributed outside the signal generator 101 (e.g., at other patient locations) while still communicating with the signal generator 101. The sensors and/or other input devices 112 can provide inputs that depend on or reflect patient state (e.g., patient position, patient posture, and/or patient activity level), and/or inputs that are patient-independent (e.g., time). Still further details are included in U.S. Patent No. 8,355,797, incorporated herein by reference in its entirety.

In some embodiments, the signal generator 101 and/or signal delivery devices 109 can obtain power to generate the therapy signals from an external power source 103. In one embodiment, for example, the external power source 103 can by-pass an implanted signal generator and generate a therapy signal directly at the signal delivery devices 109 (or via signal relay components). The external power source 103 can transmit power to the implanted signal generator 101 and/or directly to the signal delivery devices 109 using electromagnetic induction (e.g., RF signals). For example, the external power source 103 can include an external coil 104 that communicates with a corresponding internal coil (not shown) within the implantable signal generator 101, signal delivery devices 109, and/or a power relay component (not shown). The external power source 103 can be portable for ease of use.

In another embodiment, the signal generator 101 can obtain the power to generate therapy signals from an internal power source, in addition to or in lieu of the external power source 103. For example, the implanted signal generator 101 can include a non-rechargeable battery or a rechargeable battery to provide such power. When the internal power source includes a rechargeable battery, the external power source 103 can be used to recharge the battery. The external power source 103 can in turn be recharged from a suitable power source (e.g., conventional wall power).

During at least some procedures, an external stimulator or trial modulator 105 can be coupled to the signal delivery devices 109 during an initial procedure, prior to implanting the signal generator 101. For example, a practitioner (e.g., a physician and/or a company representative) can use the trial modulator 105 to vary the modulation parameters provided to the signal delivery devices 109 in real time, and select optimal or particularly efficacious parameters. These parameters can include the location from which the electrical signals are emitted, as well as the characteristics of the electrical signals provided to the signal delivery devices 109. In some embodiments, input is collected via the external stimulator or trial modulator and can be used by the clinician to help determine what parameters to vary. In a typical process, the practitioner uses a cable assembly 120 to temporarily connect the trial modulator 105 to the signal delivery devices 109. The practitioner can test the efficacy of the signal delivery devices 109 in an initial position. The practitioner can then disconnect the cable assembly 120 (e.g., at a connector 122), reposition the signal delivery devices 109, and reapply the electrical signals. This process can be performed iteratively until the practitioner obtains the desired position for the signal delivery devices 109. Optionally, the practitioner may move the partially implanted signal delivery devices 109 without disconnecting the cable assembly 120. Furthermore, in some embodiments, the iterative process of repositioning the signal delivery devices 109 and/or varying the therapy parameters may not be performed.

The signal generator 101, the lead extension 102, the trial modulator 105 and/or the connector 122 can each include a receiving element 113. Accordingly, the receiving elements 113 can be patient implantable elements, or the receiving elements 113 can be integral with an external patient treatment element, device or component (e.g., the trial modulator 105 and/or the connector 122). The receiving elements 113 can be configured to facilitate a simple coupling and decoupling procedure between the signal delivery devices 110, the lead extension 102, the pulse generator 101, the trial modulator 105 and/or the connector 122. The receiving elements 113 can be at least generally similar in structure and function to those described in U.S. Patent Application Publication No. 2011/0071593, incorporated by reference herein in its entirety.

After the signal delivery devices 109 are implanted, the patient 190 can receive therapy via signals generated by the trial modulator 105, generally for a limited period of time. During this time, the patient wears the cable assembly 120 and the trial modulator 105 outside the body. Assuming the trial therapy is effective or shows the promise of being effective, the practitioner then replaces the trial modulator 105 with the implanted signal generator 101, and programs the signal generator 201 with therapy programs selected based on the experience gained during the trial period. Optionally, the practitioner can also replace the signal delivery devices 110. Once the implantable signal generator 101 has been positioned within the patient 190, the therapy programs provided by the signal generator 101 can still be updated remotely via a wireless physician's programmer (e.g., a physician's laptop, a physician's remote or remote device, etc.) 117 and/or a wireless patient programmer 106 (e.g., a patient's laptop, patient's remote or remote device, etc.). Generally, the patient 190 has control over fewer parameters than does the practitioner. For example, the capability of the patient programmer 106 may be limited to starting and/or stopping the signal generator 101, and/or adjusting the signal amplitude. The patient programmer 106 may be configured to accept pain relief input as well as other variables, such as medication use.

In any of the foregoing embodiments, the parameters in accordance with which the signal generator 101 provides signals can be modulated during portions of the therapy regimen. For example, the frequency, amplitude, pulse width, duty cycle, and/or signal delivery location can be adjusted in accordance with a pre-set therapy program, patient and/or physician inputs, and/or in a random or pseudorandom manner. Such parameter variations can be used to address a number of potential clinical situations, including changes in the patient's perception of pain, changes in the preferred target neural population, and/or patient accommodation or habituation. Certain aspects of the foregoing systems and methods may be simplified or eliminated in particular embodiments of the present disclosure. Further aspects of these and other expected beneficial results are detailed in U.S. Patent No. 8,712,533, U.S. Patent Application Publication Nos. 2009/0204173 and 2013/0066411, and U.S. Patent Application Publication No. 2010/0274317, which are incorporated herein by reference in their entireties.

In particular embodiments, the systems and methods disclosed herein are applicable to "high frequency," paresthesia-free SCS systems. Such SCS systems, for example, inhibit, reduce, and/or eliminate pain via waveforms with high frequency elements or components (e.g., portions having high fundamental frequencies), generally with reduced or eliminated side effects. Such side effects can include unwanted motor stimulation or blocking, unwanted pain or discomfort, unwanted paresthesia, and/or interference with sensory functions other than the targeted pain. In a representative embodiment, a patient receives high frequency therapeutic signals with at least a portion of the therapy signal at a frequency of from about 1.5 kHz to about 100 kHz, or from about 2.5 kHz to about 100 kHz, or from about 1.5 kHz to about 50 kHz, or from about 3 kHz to about 10 kHz, or from about 3 kHz to about 20 kHz, or from about 3 kHz to about 50 kHz, or from about 5 kHz to about 15 kHz, or at frequencies of about 8 kHz, 9 kHz, or 10 kHz. These frequencies are significantly higher than the frequencies associated with standard conventional "low frequency" SCS, which are generally below 1,200 Hz, and more commonly below 100 Hz. Accordingly, stimulation at these and other representative frequencies (e.g., from about 1.5 kHz to about 100 kHz) is occasionally referred to herein as high frequency modulation or stimulation.

The amplitude of the signal can range from about 0.1 mA to about 20 mA in a particular embodiment, and in further particular embodiments, can range from about 0.5 mA to about 10 mA, or about 0.5 mA to about 4 mA, or about 0.5 mA to about 2.5 mA. In certain embodiments, the amplitude of the signal can be selected to be about 1 mA, or about 2 mA, or about 3mA, or about 4mA, or about 5mA. In other embodiments, the amplitude of the signal does exceed about 3mA, or about 4mA, or about 5mA. The amplitude of the applied signal can be ramped up and/or down. In particular embodiments, the amplitude can be increased or set at an initial level to establish a therapeutic effect, and then reduced to a lower level to save power without forsaking efficacy, as is disclosed in co-pending U.S. Patent Application Publication No. US2009/0204173, previously incorporated herein by reference. In particular embodiments, the signal amplitude refers to the electrical current level, e.g., for current-controlled systems. In other embodiments, the signal amplitude can refer to the electrical voltage level, e.g., for voltage-controlled systems. The pulse width (e.g., for just the cathodic phase of the pulses) can vary from about 10 microseconds to about 333 microseconds. In further particular embodiments, the pulse width can range from about 25 microseconds to about 166 microseconds, or from about 33 microseconds to about 100 microseconds, or from about 50 microseconds to about 166 microseconds, or from about 20 microseconds to about 50 microseconds. In some embodiments, the pulse width is about 30 microseconds, or about 50 microseconds, or about 80 microseconds. The specific values selected for the foregoing parameters can vary from patient to patient and/or from indication to indication and/or on the basis of the selected vertebral location. In addition, the methodology can make use of other parameters, in addition to or in lieu of those described above, to monitor and/or control patient therapy. For example, in cases for which the pulse generator includes a constant voltage arrangement rather than a constant current arrangement, the current values described above can be replaced with corresponding voltage values.

In particular embodiments, the selected pulse width, frequency and amplitude are based at least in part on the size of the electrode delivering the signal. For example, cylindrical contacts carried by a percutaneous lead and implanted with a 14 gauge needle can have an outer diameter of 1.35 mm. The length of each contact then determines the exposed surface area of the contact. The surface area, together with the signal frequency, amplitude, and pulse width can determine the amount of energy delivered by the contact, which is typically selected to produce safe and effective therapy. Table 1 below illustrates representative peak frequencies and pulse widths as a function of contact length, for a variety of peak amplitudes (assuming no interphase interval).

**Table 1.**

| | **15 mA** | | **10 mA** | | **6 mA** | |
|---|---|---|---|---|---|---|
| **Contact length (mm)** | Peak Freq. (kHz) | Peak PW (µsec) | Peak Freq. (kHz) | Peak PW (µsec) | Peak Freq. (kHz) | Peak PW (µsec) |
| 0.015 | 71.43 | 7 | 33.33 | 15 | 16.67 | 30 |
| 0.1 | 16.67 | 30 | 11.11 | 45 | 7.14 | 70 |
| 0.5 | 7.14 | 70 | 5.00 | 100 | 2.94 | 170 |
| 0.75 | 6.24 | 80 | 4.55 | 110 | 2.50 | 200 |
| 1.0 | 5.56 | 90 | 4.00 | 125 | 2.27 | 220 |

As shown in Table 1, the higher maximum frequencies can facilitate using smaller contacts without impinging on the ability of the therapy signal to produce highly effective pain relief without causing paresthesia. In fact, the smaller contacts can facilitate therapy with higher frequencies. In particular, several of the smaller electrodes can be electrically connected together and spaced apart to provide therapy to a greater volume of tissue when compared to a single, larger contact generally used to deliver conventional, low frequency SCS signals - and can do so while maintaining generally the same load impedance and power as are associated with the larger contacts. While Table 1 illustrates values associated with ring-shaped cylindrical contacts carried by cylindrical leads, in other embodiments, similar data apply to disc-shaped contacts carried by paddle leads.

As is also shown in Table 1, narrower or smaller pulse widths can also facilitate using smaller contacts. Accordingly, in at least some embodiments, signals are delivered to the smaller contacts at narrow pulse widths, but at lower frequencies than those described above. In particular, the frequencies can be less than 1.5 kHz. For example, in representative embodiments, the pulse width of the signal can range from about 10 microseconds to about 80 microseconds, and in further embodiments, can range from about 20 microseconds to about 50 microseconds. The frequencies at which the pulses are delivered can range from about 2 Hz to about 1500 Hz, or from about 20 Hz to about 500 Hz, or from about 40 Hz to about 120 Hz. In these embodiments (for which the frequency is less than about 1500 Hz), the pulsed electrical signal is generally expected to produce paresthesia in association with pain relief or another therapeutic outcome. For therapies without paresthesia, the parameters described above, (e.g. including pulse width parameters) can be used with frequencies greater than 1500 Hz. Accordingly, embodiments of the present technology include delivering therapy signals at narrow pulse widths (e.g., about 10 microseconds to about 80 microseconds, or about 20 microseconds to about 50 microseconds) in a frequency range of:
(a) from about 2 Hz to about 1500 Hz, or from about 20 Hz to about 500 Hz, or from about 40 Hz to about 120 Hz; or
(b) a frequency range of about 1500 Hz to about 100 kHz, including any of the subranges described above (e.g., from about 2.5 kHz to about 100 kHz, or from about 1.5 kHz to about 50 kHz, or from about 3 kHz to about 10 kHz, or from about 3 kHz to about 20 kHz, or from about 3 kHz to about 50 kHz, or from about 5 kHz to about 15 kHz, or at frequencies of about 8 kHz, 9 kHz, or 10 kHz), depending upon the embodiment.

Figure 1B illustrates the general arrangement of the representative leads 110 that can be connected to the signal generator 101. Further details of specific contact spacings for the lead are described later with reference to Figures 2A-2B. The lead 110 can have any suitable number of contacts C positioned along its length L for delivering electrical therapy to the patient. For purposes of illustration, eight contacts C, identified individually as contacts C1, C2 ... C8 are shown in Figure 1B. In operation, one or more of the contacts C is cathodic and another one or more of the contacts C is anodic. The contacts C can be individually addressable so that any contact C or combination of contacts C can operate as a cathode, and any contact C or combination of contacts C can operate as an anode. The contacts C can be electrically grouped in any of a wide variety of combinations, and individual contacts C can perform different functions (e.g., cathodic functions and/or anodic functions) at different times during the course of a therapy regimen. In any of these embodiments, each contact C can be coupled with a corresponding conductor 111 to the signal generator 101 (Figure 1 A) via a connector 115. The conductors 111 can have one or more connection points along their lengths (e.g., at a junction with the signal generator 101, and optionally at a junction with an extension). Accordingly, the circuit for a given pair of contacts C includes the contacts C, the patient tissue between the contacts C, the individual conductors 111, connection points along the conductors 111, and connection points between the conductors 111 and the signal generator 101.

### 2.0 Representative Lead Configurations

Figures 2A-2B illustrate representative leads 210a and 210b configured in accordance with embodiments of the present technology to be connected to the signal generator 101 of the system 100 described above. The leads 210a and 210b can include several features generally similar to those described above with respect to the leads 110 shown in Figure 1B. For example, the leads 210a and 210b can include any suitable number of contacts C positioned along the lead length L for delivering electrical therapy to the patient and corresponding conductors (not shown in Figures 2A-2B) for coupling the contacts C to the signal generator 101. However, the contacts C of leads 210a and 210b can be distributed, electrically connected together, and/or sized in manners specific to high frequency SCS treatment systems in accordance with certain embodiments of the present technology. The contacts C can operate as signal delivery electrodes, and in particular embodiments, are ring-shaped. For example, in some embodiments, the contacts C can be configured to completely encircle or extend around an outer perimeter of the lead.

Figure 2A illustrates a top view of the lead 210a having 12 contacts C, identified individually as contacts C1, C2 ... C12. In one aspect of this embodiment, the lead 210a can be elongated along a major or lead axis A. In general, the term elongated refers to a lead or other signal delivery element or device having a length (e.g., along the spinal cord) greater than its width. The lead 210a can have a distal portion length L (over which signal delivery contacts, including active contacts are positioned). The contacts C can be positioned to deliver modulation signals in accordance with particular embodiments of the present technology.

Three or more neighboring contacts C can be grouped together and electrically connected (as shown in Figure 2A) using a common conductor. For example, neighboring contacts C1-C4 can form a first contact group G1 connected to the same conductor 111 a. In particular embodiments, a single conductor is "daisy-chained" to multiple contacts in a group. That is, the single conductor can welded at several points W along its length to several corresponding contacts. In some embodiments, neighboring contacts C5-C8 and/or C9-C12 can also form contact groups for a total of three contact groups (shown as first, second, and third contact groups G1, G2, and G3), connected to corresponding common conductors 111 a, 111 b, 111 c, respectively. As used herein, and unless otherwise noted, "neighboring contacts" refers generally to two contacts on a signal delivery device that are closest or nearest to each other but can be separated or spaced apart by, for example, a gap. In a representative embodiment, a group can include three neighboring contacts, and in other embodiments, can include four (as shown in Figure 2A) or more contacts. The particular number of contacts selected for a given group can depend on factors that include the size of the target neural population, the target charge to be delivered, and/or the target charge delivery rate. In particular embodiments, it is expected that groups of three or more neighboring contacts will provide the desired signal delivery characteristics, and differ from at least some devices by providing a greater spatial coverage (e.g., than is available with two neighboring electrodes) without requiring a significant increase in current.

A further advantage of dividing what was one large electrode into a group of smaller electrodes is that the lead (or other signal delivery device) on which the electrodes are placed, can be more versatile. In particular, the lead can flex through sharper curves, making it easier to deploy and position the signal delivery device in the small volumes typical of the epidural space.

Pulses can be applied to the group(s) of contacts in accordance with several different arrangements. For example pulses provided to one group can be interleaved with pulses applied to another, or the same signal can be rapidly switched from one group to the other. In other embodiments, the signals applied to individual contacts, pairs of contacts, and/or contacts in different groups can be multiplexed in other manners. As used herein, the term "multiplex" refers to signals (anodic and/or cathodic phase) that can delivered to any individual conductor at any point in time. In other embodiments, more than three contact groups can be formed on the lead 210a. In yet other embodiments, fewer than three contact groups can be formed on the lead 210a.

Each of the contacts C can have an appropriately selected length, e.g., in the range of from about 0.015 mm to about 3 mm, and in particular embodiments, from about 0.5 mm to about 2.5 mm. In more particular embodiments, the length can be selected in the range of from about 0.75 mm to about 1.5 mm. In other embodiments, the contact lengths are less than 3 mm. Three or more neighboring contacts on a given lead can have equal or generally equal lengths. For example, contacts C1-C4 forming contact group G1 can each have a length of or about 0.75 mm.

Further, neighboring contacts of a group of three or more contacts C can be uniformly spaced apart. For example, immediately neighboring contacts of the first contact group G1 can be uniformly spaced apart (e.g., closest edge to closest edge) by a distance S1. In a representative embodiment, the distance S1 can range from about 1 mm to about 8 mm. In another particular embodiment, the distance S1 can be from about 0.75 mm to about 2.5 mm. In another particular embodiment, the distance S1 can be from about 0.75 mm to about 1 mm. In the illustrated embodiment, the neighboring contacts of the first contact group G1 are uniformly spaced apart by a distance S1 of or about 1 mm. In other embodiments, the distance S1 between neighboring contacts can be different or non-uniform with respect to different contact pairs of a contact group as described in more detail below.

In still another representative embodiment, each of the contacts C1-C4 of the first contact group G1 have equal lengths of about 0.75 mm with neighboring contacts uniformly spaced apart by a distance S1 of about 1 mm. As described above, the contacts C1-C4 can be connected together using the same conductor. By selecting the lengths and spacing of the electrically connected contacts C1-C4 with the foregoing dimensions, the first contact group G1 has a total combined length of about 6 mm (including the 1 mm spacing between neighboring contacts). This effectively doubles the length covered by the contacts C1-C4 of the first contact group G1 when compared to a standard 3 mm long contact typically used in conventional systems. When the lead 210a is coupled to the signal generator 101 (Figure 1A), the signal generator 101 can drive generally the same power across the same or generally the same load impedance as a conventional system. In particular, because the total combined contact surface area of the connected contacts C1-C4 (with .75 mm lengths) is the same as that of a single contact having a 3 mm length, the impedance of a stimulation circuit that includes four connected contacts C1-C4 is approximately the same as circuit that includes a single 3 mm long contact, and the power required to drive the two circuits is approximately the same. Therefore, it is expected that the foregoing arrangement can maintain generally the same load impedance and power as a conventional contact arrangement but can effectively stimulate more tissue via more contacts with individually smaller surface areas, e.g., group(s) of (4) 0.75 mm long contacts electrically connected together with neighboring contacts uniformly spaced apart by 1 mm. For example, in some embodiments, more vertebral levels can be covered by the smaller, spaced apart contacts as compared to a single, larger contact resulting in more potential stimulation points for pain relief.

In other embodiments, the lead 210a can include other suitable arrangements having different contact spacings, lengths, number of contacts connected together, and/or number of groups of contacts along the length of the lead, depending upon the particular patient and indication. For example, in some embodiments, more than four contacts can be electrically connected together. In other embodiments, three or more contacts can be electrically connected together. For example, contacts C1-C3 can be electrically connected together with the same conductor. The contacts C1-C3 can have generally equal lengths within any of the ranges described above, and with neighboring contacts uniformly spaced apart by a distance S1. Further, in yet other embodiments, the contacts C of a contact group G can be spaced apart by different or non-uniform distances within any of the ranges described above. For example, the distance S1 between contacts C1 and C2 can be different from (e.g., greater or less than) the distance S1 between contacts C2 and C3 of contact group G1. In certain embodiments, the contacts C of a contact group G can be spaced apart by both uniform and non-uniform distances. For example, the distances S1 between contacts C1 and C2 and C2 and C3, respectively, can be uniform while the distance S1 between contacts C3 and C4 can be different from (e.g., greater or less than) the uniform distances S1 between contacts C1 and C2 and C2 and C3 of contact group G1.

As illustrated, the lead 210a can include three groups of four contacts electrically connected together. In other embodiments, the lead 210a can include one, two, three, four or more groups of contacts. One group of contacts can have different lengths and/or contact spacings than another group of contacts. The contacts can have lengths selected to be within any of the ranges described above. Neighboring contacts of a group of contacts can be uniformly spaced apart by any of the dimensions described above, depending upon the particular embodiment. In some embodiments, the contacts can have surface areas from about 0.15 mm² to about 0.75 mm². In some embodiments, the group(s) of contacts can be spaced apart from each other by uniform distances. In other embodiments, the group(s) of contacts can be spaced apart from each other by different distances.

Figure 2B illustrates a paddle lead 210b. The paddle lead 210b generally includes contacts C that face a single direction (e.g., out of the plane of Figure 2B), unlike the contacts described above with reference to Figure 2A, which encircle the lead 210a and are omnidirectional. The contacts C can have a strip-type shape, a circular shape, and/or other suitable (and generally flat) shapes. Certain features described above with respect to the lead 210a can be similarly applied to the paddle lead 210b. For example, the paddle lead 210b includes multiple contacts C, identified individually as contacts C1, C2 ... C12, positioned to deliver modulation signals in accordance with particular embodiments of the present technology. The contacts C can be grouped and connected together in groups G (shown as first, second, and third contact groups G1, G2, and G3). The contacts C can have equal or generally equal lengths as described above with respect to Figure 2A. Neighboring contacts C of a contact group can be spaced apart uniformly by a distance S2. In other embodiments, the distance S2 can be non-uniform or different, or can be uniform for some spacings and non-uniform for others, in a manner generally similar to that discussed above with respect to the distance S1 between contacts C of lead 210a.

In the illustrated embodiment, the contacts C can have lengths of about 0.75 mm and neighboring contacts can be uniformly spaced apart by the distance S2 of about 1 mm. Further, three or more contacts, e.g., contacts C1-C4, can be electrically connected together using the same conductor (not shown in Figure 2B). In some embodiments, the contacts C can extend entirely or generally entirely across width W of the paddle lead 210b. This arrangement is expected to remove the need for separate upper and lower contacts on the paddle lead 210b (when oriented as shown in Figure 2B). Additionally, this arrangement is expected to improve (e.g., increase) charge distribution not only along the major or lead axis A in a direction parallel to the distal portion length L of the paddle lead 210b but also in a direction parallel to the width W of the paddle lead 210b, when compared to conventionally spaced and sized contacts of conventional systems. This allows the groups G of contacts C to cover more tissue and/or more vertebral levels.

One feature of at least some of the foregoing embodiments is that high frequency SCS signal delivery devices can be provided with contacts smaller (e.g., having smaller lengths, surface areas, and/or volumes) than those typically used to deliver conventional, low frequency SCS signals for activating paresthesia. Advantageously, it is expected that the smaller contacts, which are electrically connected together and spaced uniformly apart (or non-uniformly apart in some embodiments), can provide therapy to a greater volume of tissue when compared to a single, larger contact generally used to deliver conventional, low frequency SCS signals, while maintaining generally the same load impedance and power.

Generally, devices delivering low frequency SCS signals use larger contacts (e.g., having larger lengths, surface areas, and/or volumes) than those described herein with respect to certain embodiments. Typically, higher electric charge and wider pulse widths are expected for devices delivering low frequency SCS signals in order to activate paresthesia for efficacious treatment when compared with the high frequency SCS signal delivery devices described herein. For example, it is expected that devices delivering low frequency SCS signals typically do not use pulse widths below about 80 microseconds for direct nerve stimulation and below about 50 or 60 microseconds when stimulating the epidural space. Based on strength-duration curves (e.g., as known in the art), these wider pulse widths correspond to relatively higher electric charge to activate paresthesia for efficacious treatment. Decreasing the size of the contacts past a minimum size for such devices can lead to increased charge densities unsafe for treatment due to the required higher electric charge of such devices and smaller-sized contacts. Further, the issue of unsafe charge densities can be exacerbated when using multiple contacts spaced apart and electrically connected together as the charge can fractionate and be unevenly split or divided among the contacts such that the charge density is much higher at a single contact compared to the rest of the contacts.

Such charge density concerns are not expected to be an issue when delivering paresthesia-free, high frequency SCS signals. High frequency SCS signals can generally be delivered at lower pulse widths and at lower electric charge for efficacious, paresthesia-free treatment when compared with conventional, low frequency SCS signals. Therefore, in certain embodiments, it is expected that high frequency SCS signals can be delivered efficaciously and safely using smaller contacts electrically connected together as described herein.

### 3.0 Further Embodiments

In accordance with certain embodiments of the present technology, a patient therapy system includes an implantable pulse generator programmed to direct a pulsed electrical signal at a frequency from about 1.5 kHz to about 100 kHz. The patient therapy system includes at least one implantable signal delivery device having a proximal portion and a distal portion. The proximal portion is configured to be coupled with the implantable pulse generator. The distal portion includes multiple electrical contacts positioned to deliver the pulsed electrical signal to a target neural population of a patient when implanted. At least three neighboring electrical contacts of the multiple electrical contacts have equal lengths less than 3 mm. The at least three neighboring electrical contacts are uniformly spaced apart by a distance from about 1 mm to about 8 mm.

In some embodiments, the at least three neighboring electrical contacts completely encircle the implantable signal delivery device. In some embodiments, electrical contacts are electrically connected to a common conductor to receive a common electrical signal. In some embodiments, the three neighboring electrical contacts are electrically connected together and are distributed to cover more length along the signal delivery device than a single electrical contact having a length equal to a combined length of the three neighboring electrical contacts and a surface area equal to a combined total surface area of the three neighboring electrical contacts. In some embodiments, the multiple electrical contacts include at least four neighboring electrical contacts that are electrically connected together and have equal lengths of about 0.75 mm. The at least four neighboring electrical contacts are uniformly spaced apart by a distance of about 1 mm.

In accordance with another embodiment of the present technology, a patient therapy system includes at least one implantable signal delivery device having a proximal portion and a distal portion. The proximal portion is configured to be coupled with an implantable pulse generator to direct a pulsed electrical signal at a frequency from about 1.5 kHz to about 100 kHz. The distal portion includes multiple electrical contacts for delivering the pulsed electrical signal to a target neural population of a patient when implanted. At least three neighboring electrical contacts of the multiple electrical contacts are electrically connected together and have equal lengths less than 3 mm. The at least three neighboring electrical contacts are uniformly spaced apart by a distance from about 1 mm to about 8 mm.

In yet another embodiment of the present technology, a method for treating a patient includes applying a pulsed electrical signal to a patient via at least one implantable signal delivery device that includes at least three neighboring electrical contacts at a distal portion thereof. The at least three neighboring electrical contacts are electrically connected together and have equal lengths less than 3 mm. The at least three neighboring electrical contacts are uniformly spaced apart by a distance from about 1 mm to about 8 mm. The signal has a frequency from about 1.5 kHz to about 100 kHz.

In certain embodiments, the method includes applying the pulsed electrical signal to more tissue than a single electrical contact having a length equal to a combined length of the three neighboring electrical contacts and a surface area equal to a combined total surface area of the three neighboring electrical contacts. In some embodiments, the frequency is from about 3 kHz to about 20 kHz.

In another embodiment, a method for selecting a size and spacing of a plurality of electrically connected electrical contacts at an implantable signal delivery device, for applying a pulsed electrical signal to a patient having a frequency from about 1.5 kHz to about 100 kHz includes selecting a size of at least three neighboring electrical contacts such that the at least three neighboring electrical contacts have equal lengths of less than 3 mm. The method includes spacing the at least three neighboring electrical contacts by a uniform distance from about 1 mm to about 8 mm. The method further includes electrically connecting the at least three neighboring electrical contacts of the plurality of electrical contacts. In other embodiments, the contacts can have other suitable spacings (uniform or non-uniform) and/or other dimensions.

From the foregoing, it will be appreciated that specific embodiments of the present technology have been described herein for purposes of illustration, but that various modifications can be made without deviating from the present technology. For example, while certain aspects of the technology were described above in the context of spinal cord modulation to treat chronic pain, similar techniques can be used in other embodiments to treat pain in other areas for therapy applied to other neural populations and/or for other patient conditions.

The following clauses describe embodiments or features of embodiments which can be combined with one another either separately or in any combination of such features and/or embodiments.

A patient therapy system, comprising:
an implantable pulse generator programmed to direct a pulsed electrical signal at a frequency from about 1.5 kHz to about 100 kHz; and
at least one implantable signal delivery device having a proximal portion and a distal portion, the proximal portion being configured to be coupled with the implantable pulse generator, the distal portion having multiple electrical contacts positioned to deliver the pulsed electrical signal to a target neural population of a patient when implanted,
wherein at least three neighboring electrical contacts of the multiple electrical contacts have equal lengths of less than 3 mm, and wherein the at least three neighboring electrical contacts are uniformly spaced apart by a distance from about 1 mm to about 8 mm from each other.

A patient therapy system wherein at least three neighboring electrical contacts completely encircle the signal delivery device.

A patient therapy system wherein at least one implantable signal delivery device includes a paddle lead.

A patient therapy system wherein at least three neighboring electrical contacts are electrically connected to a common conductor to receive a common electrical signal.

A patient therapy system wherein at least three neighboring electrical contacts have equal lengths from about 0.015 mm to about 2 mm.

A patient therapy system wherein at least three neighboring electrical contacts have equal lengths of about 0.75 mm.

A patient therapy system wherein at least three neighboring electrical contacts are uniformly spaced apart by a distance of about 1 mm.

A patient therapy system wherein an implantable pulse generator is programmed to direct the electrical signal at a frequency of about 10 kHz.

A patient therapy system wherein at least three neighboring electrical contacts are electrically connected together and are distributed to cover more length along a signal delivery device than a single electrical contact having a length equal to a combined length of at least three neighboring electrical contacts and a surface area equal to a combined total surface area of at least three neighboring electrical contacts.

A patient therapy system wherein multiple electrical contacts include at least four neighboring electrical contacts that are electrically connected together and have equal lengths of about 0.75 mm and wherein the at least four neighboring electrical contacts are uniformly spaced apart by a distance of about 1 mm.

A patient therapy system wherein a signal delivery device is elongated along an axis, and wherein the lengths of multiple electrical contacts are measured along the axis.

A patient therapy system comprising a common electrical conductor carried by the signal delivery device, and wherein at least three neighboring electrical contacts are connected directly to the common electrical conductor.

A patient therapy system, comprising:
at least one implantable signal delivery device having a proximal portion and a distal portion, the proximal portion being configured to be coupled with an implantable pulse generator to direct a pulsed electrical signal at a frequency from about 1.5 kHz to about 100 kHz, the distal portion having multiple electrical contacts for delivering the pulsed electrical signal to a target neural population of a patient when implanted,
wherein at least three neighboring electrical contacts of the multiple electrical contacts are electrically connected together and have equal lengths less than 3 mm and wherein the at least three neighboring electrical contacts are uniformly spaced apart by a distance from about 1 mm to about 8 mm.

A patient therapy system comprising an implantable pulse generator.

A patient therapy system wherein an implantable pulse generator is programmed to direct the electrical signal at a frequency of about 10 kHz.

A patient therapy system, comprising:
an implantable pulse generator programmed to direct a pulsed electrical at a pulse width from about 10 microseconds to about 80 microseconds; and
at least one implantable signal delivery device having a proximal portion and a distal portion, the proximal portion being configured to be coupled with the implantable pulse generator, the distal portion having multiple electrical contacts positioned to deliver the pulsed electrical signal to a target neural population of a patient when implanted,
wherein at least three neighboring electrical contacts of the multiple electrical contacts have equal lengths of less than 3 mm, and are uniformly spaced apart by a distance from about 1 mm to about 8 mm from each other.

A patient therapy system wherein the pulse width has a value of from about 10 microseconds to about 80 microseconds; or from about 20 microseconds to about 50 microseconds.

A patient therapy system wherein the pulsed electrical signal has a frequency of from about 2 Hz to about 1,500 Hz.

A patient therapy system wherein the pulsed electrical signal has a frequency of from about 20 Hz to about 500 Hz.

A patient therapy system wherein the pulsed electrical signal has a frequency of from about 40 Hz to about 120 Hz.

A patient therapy system wherein at least one implantable signal delivery device includes a paddle lead.

A patient therapy system wherein at least three neighboring electrical contacts completely encircle the signal delivery device.

A patient therapy system wherein at least three neighboring electrical contacts are electrically connected to a common conductor to receive a common electrical signal.

A patient therapy system wherein at least three neighboring electrical contacts have equal lengths from about 0.015 mm to about 2 mm.

A patient therapy system wherein at least three neighboring electrical contacts have equal lengths of about 0.75 mm.

A patient therapy system wherein at least three neighboring electrical contacts are uniformly spaced apart by a distance of about 1 mm.

A patient therapy system wherein an implantable pulse generator is programmed to direct the electrical signal at a frequency of about 10 kHz.

A patient therapy system wherein at least three neighboring electrical contacts are electrically connected together and are distributed to cover more length along a signal delivery device than a single electrical contact having a length equal to a combined length of the at least three neighboring electrical contacts and a surface area equal to a combined total surface area of the at least three neighboring electrical contacts.

A patient therapy system wherein at least three neighboring electrical contacts have equal lengths of less than 3 mm, and are uniformly spaced apart by a distance from about 1 mm to about 8 mm from each other.

A patient therapy system wherein multiple electrical contacts include at least four neighboring electrical contacts that are electrically connected together and have equal lengths of about 0.75 mm and wherein the at least four neighboring electrical contacts are uniformly spaced apart by a distance of about 1 mm.

A patient therapy system wherein a signal delivery device is elongated along an axis, and wherein the lengths of the multiple electrical contacts are measured along the axis.

A patient therapy system comprising a common electrical conductor carried by a signal delivery device, and wherein at least three neighboring electrical contacts are connected directly to the common electrical conductor.

A method for treating a patient, comprising:
applying a pulsed electrical signal to a patient via at least one implantable signal delivery device that includes at least three neighboring electrical contacts at a distal portion thereof, wherein the at least three neighboring electrical contacts are electrically connected together and have equal lengths less than 3 mm, wherein the at least three neighboring electrical contacts are uniformly spaced apart by a distance from about 1 mm to about 8 mm, and wherein the signal has a frequency from about 1.5 kHz to about 100 kHz.

A method wherein applying a pulsed electrical signal includes applying the signal to more tissue than a single electrical contact having a length equal to a combined length of the three neighboring electrical contacts and a surface area equal to a combined total surface area of the three neighboring electrical contacts.

A method wherein the frequency is from about 3 kHz to about 20 kHz.

A method for selecting a size and spacing of a plurality of electrically connected electrical contacts of an implantable signal delivery device, for applying a pulsed electrical signal to a patient at a frequency from about 1.5 kHz to about 100 kHz, the method comprising:
selecting a size of at least three neighboring electrical contacts such that the at least three neighboring electrical contacts have equal lengths of less than 3 mm;
spacing the at least three neighboring electrical contacts by a uniform distance from about 1 mm to about 8 mm; and
electrically connecting the at least three neighboring electrical contacts of the plurality of electrical contacts.

A method wherein selecting the size of at least three neighboring electrical contacts includes selecting the size of the contacts to be 0.75 mm long.

A method wherein spacing neighboring electrical contacts includes spacing the neighboring electrical contacts by a uniform distance of about 1 mm.

A method wherein selecting the size of at least three neighboring electrical contacts includes selecting the size of four neighboring contacts such that four neighboring electrical contacts have equal lengths of about 0.75 mm and a uniform spacing of about 1 mm.

Certain aspects of the foregoing technology described in the context of particular embodiments can be combined or eliminated in other embodiments. For example, the contact distribution and sizing described herein can be combined with contacts with typical sizes and distributions located at a different portion of the lead. Additional representative therapy devices and methods are disclosed in U.S. Patent No. 8,712,533, U.S. Patent Publication 2009/0204173, and U.S. Patent Publication 2013/0066411, all of which were previously incorporated herein by reference. Further, while advantages associated with certain embodiments have been described in the context of those embodiments, other embodiments can also exhibit such advantages, and not all embodiments need necessarily exhibit such advantages to fall within the scope of the present technology.

To the extent any of the materials incorporated by reference herein conflict with the present disclosure, the present disclosure controls.

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be utilised for realising the invention in diverse forms thereof.

## Claims

1. A patient therapy system, comprising:
at least one implantable signal delivery device having a proximal portion and a distal portion and an axis from the proximal portion to the distal portion, the proximal portion being configured to be coupled with an implantable pulse generator programmed to direct a pulsed electrical signal at a frequency from 1.5 kHz to 100 kHz , the distal portion having multiple electrical contacts positioned to deliver the pulsed electrical signal to a target neural population of a patient when implanted,
wherein at least three neighboring electrical contacts of the multiple electrical contacts have equal lengths of less than 3 mm along the axis, and wherein the at least three neighboring electrical contacts are uniformly spaced apart along the axis by a distance from 1 mm to 8 mm from each other.

2. The patient therapy system of claim 1, further comprising the implantable pulse generator.

3. The patient therapy system of claim 1 wherein the at least three neighboring electrical contacts completely encircle the signal delivery device.

4. The patient therapy system of claim 1 wherein the at least one implantable signal delivery device includes a paddle lead.

5. The patient therapy system of claim 1 wherein the at least three neighboring electrical contacts are electrically connected to a common conductor to receive a common electrical signal.

6. The patient therapy system of claim 1 wherein the at least three neighboring electrical contacts have equal lengths from 0.015 mm to 2 mm.

7. The patient therapy system of claim 6 wherein the at least three neighboring electrical contacts have equal lengths of 0.75 mm.

8. The patient therapy system of claim 1 wherein the at least three neighboring electrical contacts are uniformly spaced apart by a distance of 1 mm.

9. The patient therapy system of claim 1 wherein the implantable pulse generator is programmed to direct the electrical signal at a frequency of from 1.5 kHz to 100 kHz, or from 2.5 kHz to 100 kHz, or from 1.5 kHz to 50 kHz, or from 3 kHz to 10 kHz, or from 3 kHz to 20 kHz, or from 3 kHz to 50 kHz, or from 5 kHz to 15 kHz, or at frequencies of 8 kHz, 9 kHz, or 10 kHz..

10. The patient therapy system of claim 1 wherein the at least three neighboring electrical contacts are electrically connected together and are distributed to cover more length along the signal delivery device than a single electrical contact having a length equal to a combined length of the at least three neighboring electrical contacts and a surface area equal to a combined total surface area of the at least three neighboring electrical contacts.

11. The patient therapy system of claim 1 wherein the multiple electrical contacts include at least four neighboring electrical contacts that are electrically connected together and have equal lengths of 0.75 mm and wherein the at least four neighboring electrical contacts are uniformly spaced apart by a distance of 1 mm.

12. The patient therapy system of claim 1 wherein the signal delivery device is elongated along the axis, and wherein the lengths of the multiple electrical contacts are measured along the axis.

13. The patient therapy system of claim 1, further comprising a common electrical conductor carried by the signal delivery device, and wherein the at least three neighboring electrical contacts are connected directly to the common electrical conductor.

14. The patient therapy system of claim 1, wherein the implantable pulse generator is programmed to direct a pulsed electrical signal at a pulse width from 10 microseconds to 80 microseconds; or from 20 microseconds to 50 microseconds.
